# EUROPÄISCHE PATENTANMELDUNG

(11) **EP 1 224 913 A2**
(43) Veröffentlichungstag der Anmeldung: **24.07.2002**
(21) Anmeldenummer: 01130671.9
(22) Anmeldetag: 21.12.2001
(51) Int. Cl.: A61B 17/16, A61C 1/07, A61C 1/14, A61C 3/16

(54) **Maschineller Impulsgeber für dentalmedizinische oder medizinische Zwecke**

(30) Priorität: 23.01.2001 DE 10102985
(71) Anmelder: KALTENBACH & VOIGT GmbH & Co., 88400 Biberach/Riss (DE)
(72) Erfinder: Heckenberger, Hans, 88400 Biberach (DE)
(74) Vertreter: Schmidt-Evers, Jürgen, Dipl.-Ing.

(57) **Zusammenfassung**

Die Erfindung betrifft einen maschinellen Impulsgeber (31) für dentalmedizinische oder medizinische Zwecke mit einem beweglichen Impulskopf (46a), der bei Auslösung eines Impulses von einer Bereitschaftsposition schlagartig in eine bestimmte Wirkrichtung (R1, R2) bewegbar ist. Um die Handhabbarkeit des Impulsgebers (31) zu verbessern, ist der Impulskopf (46a) bei Auslösung eines Impulses ebenso schlagartig in die entgegengesetzte Richtung (R1, R2) bewegbar, wobei die Richtung (R1, R2) vorher einstellbar ist.

## Beschreibung

Die Erfindung bezieht sich auf einen maschinellen Impulsgeber nach dem Oberbegriff des Anspruchs 1.

Ein maschineller Impulsgeber dieser Art wird von der Anmelderin unter der Gerätebezeichnung CORONAflex® 205 vertrieben und ist somit Stand der Technik. Dieser vorbekannte Impulsgeber ist geeignet, mit einem Impulskopf schlagartig auf einen mit dem Impulskopf koppelbaren Kronenzieher einzuwirken und eine schlagartige Bewegung auf den Kronenzieher zu übertragen.

Der bekannte Impulsgeber ist auf dem Markt eingeführt und hat sich bewährt. Er ist in ein sogenanntes Handstück integriert, das einen sich gerade erstreckenden Handstückschaft aufweist, von dem sich ein Handgriff quer erstreckt, an dem der Behandler das Handstück ergreifen und handhaben kann. Der Impulskopf überragt das freie Ende des Handstückschaftes in einer einseitigen, zur dem Handgriff abgewandten Seite hin versetzten Position. Der Impulskopf ist am freien Ende eines sich etwa parallel zur Längsmittelachse des Handstückschaftes erstreckenden Impulsgeberhebels angeordnet, der in einem Hebelgelenk um eine bezüglich derLängsmittelachse des Handstückschaftes quer und seitlich versetzt verlaufende Gelenkachse schwenkbar gelagert ist und mit einem bezüglich des Gelenks quer abstehenden Impulsnehmerhebel einen Winkelhebel bildet, der in einer vorderseitig offenen Gelenkausnehmung des Hebelgelenks im Handstückschaft schwenkbar gelagert ist. Die für eine Impulsbewegung des Impulskopfes erforderliche schlagartige Bewegung des Winkelhebels wird durch einen Schlagkolben erzeugt, der in einem sich vom Impulsnehmerhebel nach hinten erstreckenden Führungskanal hin und her verschiebbar gelagert ist und aus einer hinteren Startposition durch Druckluft gegen den Impulsnehmerhebel geschossen wird. Zur Auslösung des Schusses ist ein am Handstück angeordnetes Betätigungselement vorgesehen, mit dem direkt oder mittelbar ein Ventil betätigbar ist, das die den Schlagkolben beaufschlagende Druckluftzufuhr steuert.

Bei diesem vorbekannten Impulsgeber ist die Wirkrichtung des Impulskopfes bezüglich der Längsmittelachse des Handstückschaftes radial nach außen gerichtet. Es ist deshalb erforderlich, den Impulsgeber jeweils so zu positionieren, daß die Wirkrichtung in die von der Zahnwurzel wegweisenden Achsrichtung des Zahns gerichtet ist. Wenn der Schlagkolben gegen die Impulsnehmerhebel schlägt, wird die übertragene Impulsenergie durch das Gelenk auf den Impulskopf übertragen, der die Impulsenergie mittelbar über den Kronenzieher auf die Zahnkrone weiterleitet. Wenn ein Schuß in einer Position des Impulskopfes ausgelöst wird, in der dieser durch den Kronenzieher nicht mit dem zu beaufschlagenden Teil verbunden ist, ist die Schlagenergie des Schlagkolbens zu einem Großteil von dem Schwenkgelenk aufzunehmen.

Der Erfindung liegt die Aufgabe zugrunde, bei einem Impulsgeber der eingangs angegebenen Art dessen Handhabbarkeit zu verbessern.

Diese Aufgabe wird durch die Merkmale des Anspruchs 1 gelöst. Vorteilhafte Weiterbildungen der Erfindung sind in den Unteransprüchen beschrieben.

Der erfindungsgemäße Impulskopf ist zur Auslösung eines Impulses ebenso schlagartig wahlweise in eine von zwei einander entgegengesetzten Wirkrichtungen bewegbar, wobei die gewünschte Wirkrichtung vorher einstellbar ist. Hierdurch ist der Impulsgeber geeignet, jeweils in eine von beiden einander entgegengesetzten Wirkrichtungen eingesetzt zu werden. Das bedeutet in der Praxis, daß der Impulsgeber aus ein und derselben Arbeitsstellung heraus so einstellbar ist, daß er in eine wahlweise von beiden einander entgegengesetzten Wirkrichtungen eingesetzt werden kann. Dies ist insbesondere an Behandlungsstellen vorteilhaft, die sich in einem beengten Raum befinden, wie es z. B. im Mundraum des menschlichen oder tierischen Körpers der Fall ist. Außerdem ist der erfindungsgemäße Impulsgeber geeignet, ohne eine Veränderung seiner Position für Arbeiten sowohl am Unterkiefer als auch am Oberkiefer eingesetzt zu werden.

Zur Voreinstellung der gewünschten Wirkrichtung kann eine z. B. manuell betätigbare Einstellvorrichtung vorgesehen sein. Es ist vorteilhaft, eine solche Einstellvorrichtung im vorderen Endbereich des Handstückschaftes anzuordnen, wo sie für die Bedienungsperson handhabungsfreundlich zugänglich ist.

Für die erfindungsgemäße Ausgestaltung eignet sich eine solche Weiterbildung vorteilhaft, bei der der Impulskopf einen zweiten Auftreffbereich für den Schlagkolben aufweist, der - vom Schlagkolben aus gesehen - in Bezug auf die Schwenkachse gegenüberliegend zu dem ersten Auftreffbereich angeordnet ist. Hierdurch bildet der Impulskopf eine um die Schwenkachse schwenkbare Wippe, so daß sich der Impulskopf in die eine oder in die andere Richtung schwenkt, je nach dem, welcher Auftreffbereich näher am Schlagkolben ist, bevor dieser auf den Impulskopf auftrifft.

Ein vorteilhaftes Ausführungsbeispiel hierfür ist ein Impulsschwenkteil mit im Sinne einer Wippe einander entgegengesetzt angeordneten Impulsnehmerhebeln, die bezüglich des Impulsgeberhebels und des Schwenkgelenks sich in einander entgegengesetzte Richtungen erstrecken und dadurch die Wippe bilden. Bei einer solchen Ausgestaltung ist eine wahlweise Wirkrichtung der beiden möglichen einander entgegengesetzt gerichteten Wirkrichtungen dadurch möglich, daß der Impulskopf an der Behandlungsstelle in die der gewünschten Wirkrichtung entgegengesetzte Richtung bewegt wird. Infolge dessen wird der zugehörige Wippenarm in eine Position gebracht, in der er dem Schlagkolben bei dessen Auftreffen näher liegt als der andere Wippenarm und somit die Schlagenergie übernimmt. In der Praxis wird hierzu der Impulskopf in einfacher Weise gegen den zu behandelnden Gegenstand, z. B. ein Zahn, bewegt oder gedrückt. Hierdurch ist ein vorteilhaftes Arbeitsverfahren des Impulsgebers geschaffen. Für eine entgegengesetzte Wirksamkeit läßt sich der Impulskopf in die entgegengesetzte Wirkrichtung bewegen bzw. drücken.

Insbesondere dann, wenn der Impulskopf durch das Werkzeug nicht mittelbar mit dem zu behandelnden Körper verbunden ist, wird bei einer z. B. ungewollten Auslösung des Schlagkolbens dessen Energie zu einem Großteil vom Schwenkgelenk aufgenommen. Hierdurch können der Impulsschwenkhebel und/oder das Schwenkgelenk beschädigt werden oder zumindest die Lebensdauer herabgesetzt werden.

Der Erfindung liegt deshalb die Aufgabe zugrunde, einen Impulsgeber der im Oberbegriff des Anspruchs 16 angegebenen Art so auszugestalten, daß die Beaufschlagung des Impulskopfes bei unsachgemäßer Auslösung des Impulses vermindert oder verhindert wird.

Diese Aufgabe wird durch die Merkmale des Anspruchs 16 gelöst. Vorteilhafte Weiterbildungen der Erfindung sind in zugehörigen Unteransprüchen beschrieben.

Bei der erfindungsgemäßen Ausgestaltung nach Anspruch 16 ist dem Impulsgeber eine Dämpfungsvorrichtung so zugeordnet, daß die Impulsauslösung nur dann auf den Impulskopf voll übertragen wird, wenn dies erwünscht ist. Dies ist z. B. dann der Fall, wenn der Impulskopf vorher richtig eingestellt worden ist bzw. in seine Bereitschaftsstellung bewegt worden ist, in der der zugehörige Auftreffbereich dem Schlagkolben näher angeordnet ist als der andere Auftreffbereich.

Die Dämpfungsvorrichtung ist vorzugsweise durch ein Ventil gebildet, das die beim Vorschießen des Schlagkolbens entweichende Luft dazu ausnutzt, ein elastisches Druckpolster zu bilden.

Der erfindungsgemäße Impulsgeber eignet sich für den Antrieb bzw. eine Impulsbeaufschlagung von solchen Werkzeugen, die eine kurzzeitige schlagende Bewegung ausführen sollen, wie es z. B. zum Lösen einer Krone oder zum Setzen eines Osteotoms erforderlich ist. Hierbei ist der Impulsgeber manuell mit dem Werkzeug in Antriebsverbindung zu bringen, wobei das Werkzeug und der Impulsgeber manuell zu halten sind.

Der Erfindung liegt deshalb im weiteren die Aufgabe zugrunde, einen Impulsgeber der im Oberbegriff des Anspruchs 1 angegebenen Art so auszugestalten, daß die mit ihm durchzuführenden Arbeiten handhabungsfreundlich ausführbar sind.

Diese Aufgabe wird durch die Merkmale des Anspruchs 16 gelöst. Vorteilhafte Weiterbildungen der Erfindung sind in den zugehörigen Unteransprüchen beschrieben.

Bei der erfindungsgemäßen Ausgestaltung nach Anspruch 16 weist der Impulsgeber im vorderen Endbereich seines den Impulskopf tragenden Körpers eine Haltevorrichtung mit einer Führung für ein in seiner Achsrichtung hin und her bewegbares Werkzeug auf, insbesondere für ein Osteotom. Bei dieser Ausgestaltung ist das Werkzeug in seiner mit dem Impulskopf in Antriebsverbindung stehenden Position am Impulsgeber gehalten und geführt. Hierbei entfällt eine besondere Positionierung, Halterung und Führung des Werkzeugs an der Behandlungsstelle, wobei bei Einhandbedienung das Werkzeug mit dem Impulsgeber zur Behandlungsstelle geführt und an der Behandlungsstelle positioniert, ausgerichtet und zur Ausführung seiner Arbeit im Sinne eines Vorschubs vorbewegt werden kann. Hierdurch wird die Handhabung wesentlich vereinfacht. Dies gilt insbesondere für beengte Behandlungsstellen, wie es Mundraum des menschlichen oder tierischen Körpers der Fall ist.

In weiteren Unteransprüchen sind Merkmale enthalten, die zu einer kleinen Bauweise führen, die sich vorteilhaft an einem Handstückschaft verwirklichen lassen, eine zuverlässige Funktion langer Lebensdauer ergeben und im weiteren zur Handhabungsfreundlichkeit beitragen.

Nachfolgend werden vorteilhafte Ausgestaltungen der Erfindung anhand mehrerer Ausführungsbeispiele und Zeichnungen näher erläutert. Es zeigt
- Fig. 1: einen erfindungsgemäßen Impulsgeber mit einem Handstück in der Seitenansicht;
- Fig. 2: den Impulsgeber im axialen Schnitt in vergrößerter Darstellung;
- Fig. 3: den Impulsgeber im axialen Schnitt in abgewandelter Ausgestaltung;
- Fig. 4: den Impulsgeber im axialen Schnitt in weiter abgewandelter Ausgestaltung;
- Fig. 5: den Impulsgeber in der Stirnansicht mit Werkzeug;
- Fig. 6: den Impulsgeber in der Stirnansicht ohne Werkzeug;
- Fig. 7: den Querschnitt VII-VII in Fig. 3 oder 4;
- Fig. 8: den Längsteilschnitt VIII-VIII in Fig. 4 in verkleinerter Ausgestaltung;
- Fig. 9: den Querschnitt IX-IX in Fig. 4;

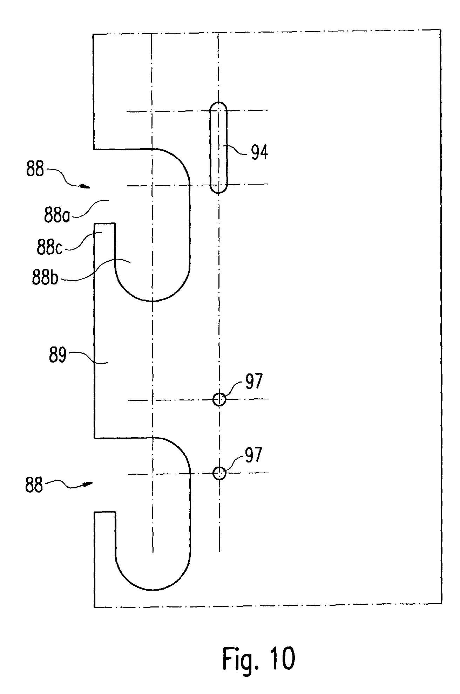

- Fig. 10: eine Bajonetthülse in ihrer Abwicklung;
- Fig. 11: einen erfindungsgemäßen Impulsgeber im axialen Schnitt in weiter abgewandelter Ausgestaltung;
- Fig. 12: den Querschnitt XII-XII in Fig. 11;
- Fig. 13: eine Exzenterhülse in der Seitenansicht;
- Fig. 14: die Exzenterhülse nach Fig. 13 in der Ansicht von links.

Der in seiner Gesamtheit mit 31 bezeichnete Impulsgeber ist in einen Handstückschaft 32 eines medizinischen oder dentalmedizinischen Handstücks 33 integriert, das einen vom sich gerade erstreckenden Handstückschaft 32 in seinem hinteren Bereich quer abstehenden Handgriff 34 aufweist, an dem das Handstück 33 zu seiner Handhabung manuell ergriffen wird.

Das Handstück 33 ist durch eine Schnellkupplung 35, insbesondere eine Steckkupplung oder eine Steck/Drehkupplung, mit einem im wesentlichen zylindrischen Kupplungszapfen 36 und einer diesen drehbar aufnehmenden Kupplungsausnehmung 37 mit einer flexiblen Versorgungsleitung 38 lösbar verbindbar, die mit einem nicht dargestellten Steuergerät verbunden ist und durch die sich Medienleitungen zur Versorgung des Handstücks 33 erstrecken, insbesondere eine Druckluftleitung 38a, die die Steckkupplung in an sich bekannter Weise abgedichtet so durchsetzt, daß das Handstück 33 in der Steck/Drehkupplung bei Gewährleistung des Durchgangs der Druckluft frei drehbar ist. Zur axialen Fixierung des Kupplungszapfens 36 in der Kupplungsausnehmung 37 ist eine Verrastungsvorrichtung 39 mit einem elastisch ausfederbaren Verrastungselement 39a vorgesehen, das beim Kuppeln selbsttätig einrastet und zum Lösen durch eine manuelle axiale Kraftausübung elastisch ausfedert und die Kupplung lösbar ist.

Beim vorliegenden Ausführungsbeispiel dient die Druckluft als Antriebsmittel für einen Schlagkolben 41, der in einem axialen Führungskanal 42 im Handstückschaft 32 aus einer hinteren Startstellung durch Druckluftbeaufschlagung nach vorne in eine Schlagstellung schießbar ist, in der er seine Aufschlag- bzw. Impulsenergie entweder direkt an ein Übertragungsglied 43 oder an ein Dämpfungsglied abgibt, das am hinteren Ende des Übertragungsglieds 43 angrenzt bzw. anliegt. Der Führungskanal 42 ist durch eine radiale Stegwand 32c in seinem vorderen Bereich geschlossen. Der Druckluftantrieb und eine zugehörige Steuerung zum Betätigen des Schlagkolbens 41 ist durch offenkundige Vorbenutzung am eingangs bereits beschriebenen Impulsgeber CORONAflex® 2005 der Anmelderin bekannt und braucht deshalb nicht weiter beschrieben zu werden.

Das Übertragungsglied 43 ist schwenkbar im Handstückschaft gelagert und durch einen T-förmigen und somit dreiarmigen Schwenkhebel 45 gebildet, der einen sich nach vorne erstreckenden Impulsgeberhebel 46 und zwei sich von dessen Fußbereich nach beiden einander gegenüberliegenden Seiten erstreckende Impulsnehmerhebel 47a, 47b aufweist. Das Schwenklager 48 mit der Schwenkachse 48a ist im für alle drei Hebel gemeinsamen Fußpunkt bezüglich der Impulsnehmerhebel 47a, 47b mittig angeordnet, wobei die Schwenkachse 48a die Längsmittelachse 32a des Handstückschafts 32 im wesentlichen schneidet. Das Schwenklager 48 ist durch einen Gelenkbolzen 49 gebildet, der die Stegwand 32c, den Schwenkhebel 45 und den Handstückschaft 32 in Bolzenbzw. Gelenklöcher durchfaßt und darin axial gesichert ist, z. B. durch eine Sicherungsschraube oder durch Preßsitz in einem der vorgenannten Teile. Zur seitlichen Begrenzung und Führung des Schwenkhebels 45 ist eine rückseitig und vorderseitig offene Lagerausnehmung 51 rechteckigen Querschnitts im Handstückschaft 32 vorgesehen, in der der Schwenkhebel 45 mit Bewegungsspiel schwenkbar ist. Am vorderen Ende des Impulsgeberhebels 46 ist ein in der Schwenkebene E vorzugsweise verdickter Impulskopf 46a ausgebildet, dessen Seitenflächen vorzugsweise zylinderabschnittförmig gerundet sind, wodurch sich konvex gerundete Impulskopfflächen 52a, 52b ergeben.

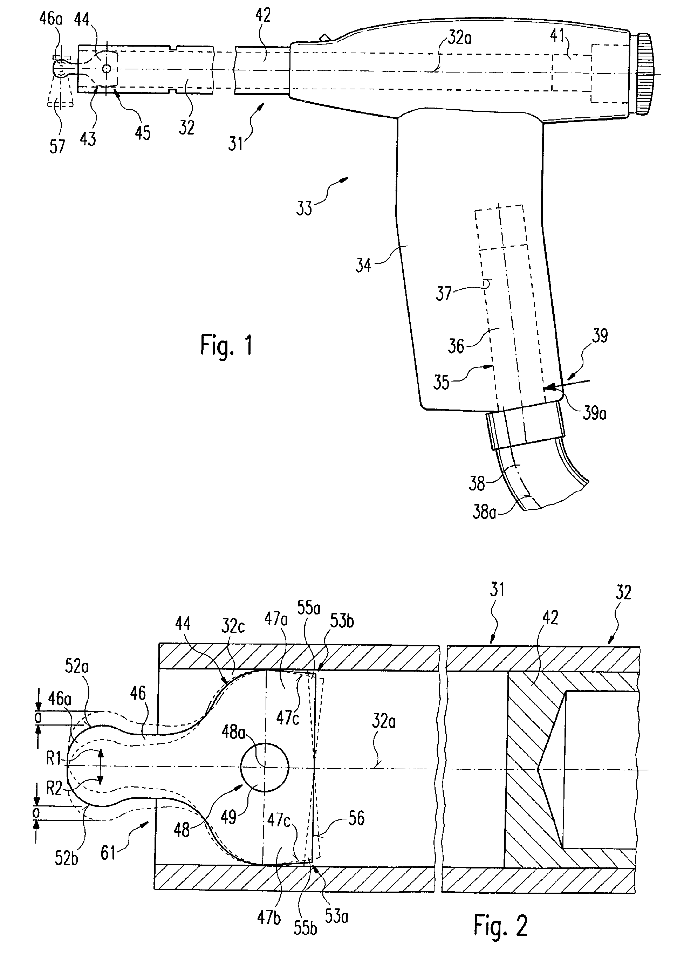

Der Schwenkhebel 45 ist um ein paar Grad hin und her schwenkbar, so daß der Impulsgeberkopf 46a in beide quer zur Handstückschaft 32 gerichtete Wirkrichtungen R1, R2 bewegbar ist. Die zugehörigen Schwenkendstellungen sich durch Anschläge begrenzt. Hierzu können z. B. die seitlichen Endflächen 47c der Impulsnehmerhebel 47a, 47b dienen, die nach hinten konvergent ausgebildet sein können und an der jeweils gegenüberliegend angeordneten Begrenzungsfläche der Lagerausnehmung 51 anschlagen können. Die Anschlagstellungen sind in Fig. 2 gestrichelt angedeutet.

Die einander gegenüberliegenden Impulsnehmerhebel 47a, 47b bilden eine um das Schwenklager 48 schwenkbare Wippe, wobei die Hebelenden 55a, 55b zwei bezüglich der Längsmittelachse 32a seitlich einander gegenüberliegend versetzt angeordnete Auftreffbereiche für den Schlagkolben 41 bilden. Es läßt sich somit ein quer zum Handstückschaft 32 wirksamer Schlagimpuls auf ein Werkzeug ausüben, z. B. auf eine in Fig. 1 andeutungsweise dargestellte Schlaufe 57 zur Lösen einer Zahnkrone, wie es in einem Prospekt der Anmelderin mit der Bezeichnung "Zahnersatz-Entfemungssystem CORONAflex® 2005" an sich beschrieben ist.

Die erfindungsgemäße Ausgestaltung ermöglicht eine Impulsabgabe mit dem Impulskopf 46a in die eine oder in die andere Wirkrichtung R1, R2. Dies ist davon abhängig, welches der beiden Hebelenden 55a, 55b dem Schlagkolben 41 näher liegt. Hierdurch ist in dem Impulsgeber 31 eine Voreinstellvorrichtung 61 integriert enthalten, die es ermöglicht, den Impulsgeber 31 wahlweise so einzustellen, daß der Impulskopf 46a in die eine oder in die andere Wirkrichtung R1, R2 wirksam ist. Diese Voreinstellung läßt sich in handhabungsfreundlicher und einfacher Weise bei der Bearbeitung des Körperteils durchführen. Um z. B. die Wirkrichtung R1 einzustellen, wird der Impulskopf 46a in seine dieser Wirkrichtung R1 entgegengesetzt Richtung R2 in eine Bereitschaftsstellung (strichpunktiert dargestellt) bewegt, vorzugsweise bis zum Anschlag 53b, so daß bei Impulsauslösung der Schlagkolben 41 auf das Hebelende 55b trifft und dadurch der Impulskopf 46a um das Maß a in die Wirkrichtung R1 in die mit durchgezogener Linie dargestellte Ausgangsstellung zurückschwenkt. Diese Voreinstellung kann in einfacher Weise dadurch erfolgen, daß mit dem Handstück 33 und einem Werkzeug, z. B. mittels der Schlaufe 57, bei einer Abstützung am Körperteil (Zahn) eine geringe Druck- oder Zugspannung, hier bei einem Kronenenlösewerkzeug eine Zugspannung, auf den Impulskopf 46 ausgeübt wird, wodurch dieser in die betreffende Bereitschaftsstellung geschwenkt wird. Bei einer Bewegung (Zug oder Druck) des Impulskopfes 46a in die entgegengesetzte Richtung, siehe R1, wird die andere Wirkrichtung R2 für den Impulskopf 46a eingestellt. Im Rahmen der Erfindung können auch andere Voreinstellvorrichtungen vorgesehen sein, z. B. mit einem zusätzlichen, hin und her beweglichen Einstellglied.

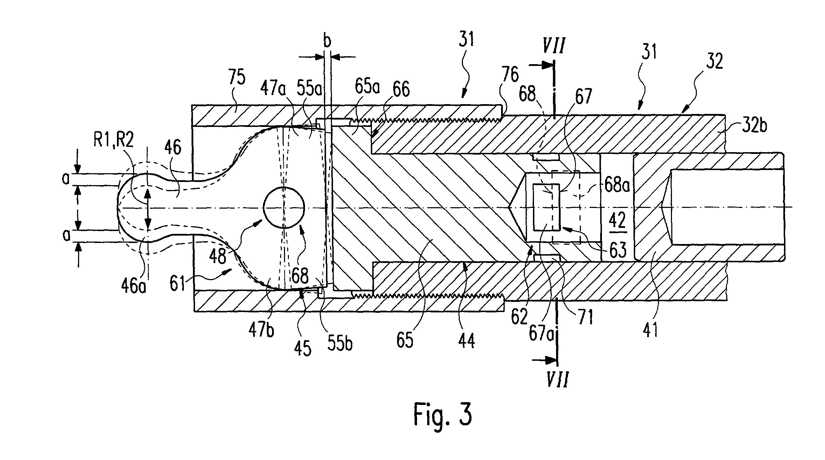

Das Ausführungsbeispiel nach Fig. 3, bei dem gleiche oder vergleichbare Teile mit gleichen Bezugszeichen versehen sind, unterscheidet sich vom vorbeschriebenen Ausführungsbeispiel durch einen unabhängigen Unterschied, nämlich dadurch, daß dem Impulsgeber 31 eine Dämpfungsvorrichtung 62 mit dem vorerwähnten Dämpfungsglied 44 zugeordnet ist, die dann in Funktion tritt, wenn der Schlagkolben 41, z. B. unbeabsichtigt, in einer Stellung des Impulskopfes 46a ausgelöst wird, in der dieser sich nicht in einer seiner Bereitschaftsstellungen befindet, nämlich in seiner Mittelstellung. In diesem Falle würde die Schlagenergie auf die Teile des Schwenklagers 48 auftreffen, ohne daß sie in den zu bearbeitenden Körper weitergeleitet werden könnte. Hierdurch könnte eine Beschädigung oder ein vergrößerter Verschleiß des Impulsgebers 31 hervorgerufen werden. Deshalb ist die z. B. pneumatische Dämpfungsvorrichtung 62 vorgesehen. Sie sperrt ein Entweichen der beim Vorschießen des Schlagkolbens 41 aus dem Führungskanal 42 an wenigstens einer Luftöffnung 63 auströmenden Luft, so daß die im Führungskanal 42 befindliche Luft ein elastisches Polster für den Schlagkolben 41 bildet. Hierzu ist ein Sperrelement vorgesehen, das im Sinne eines Ventilschiebers mit der Luftöffnung 63 zusammenwirkt und beim Ausführungsbeispiel durch einen Übertragungskolben 65 gebildet ist, der am vorderen Ende des Führungskanals 42 geringfügig axial bewegbar gelagert ist und an seinem vorderen Ende mit den Impulsnehmerhebeln 47a, 47b in Verbindung steht, vorzugsweise durch Anlage. Der axiale Hub b des Übertragungskolbens 65 ist durch einen rückseitigen Anschlag 66 so begrenzt, daß die Hebelenden 55a bzw. 55b in ihren Bereitschaftsstellungen am Übertragungskolben 65 anliegen. In dieser Stellung stehen die vorderen und hinteren, das Luftloch 63 in der Hülsenwand 32b begrenzenden Begrenzungskanten 67, 68, z. B. von wenigstens zwei einander deckenden radialen Löchern 67a, 68a in der Hülsenwand 32b und im Übertragungskolben 65 in gegenseitiger Überdeckung, wodurch die Luftöffnung 63 gebildet ist. Wenn dagegen der Impulskopf 46a in seiner mittleren Ausgangsstellung steht, wird der Übertragungskolben 65 durch den beim Vorschießen des Schlagkolbens 41 im Führungskanal 42 entstehenden Überdruck nach vorne gegen die Rückenfläche 56 verschoben, wobei die Löcher 67a, 68a außer Deckung kommen und das Luftloch 63 geschlossen wird. Hierbei erhöht sich der Luftdruck im Führungskanal 42, so daß der Schlagkolben 41 wirksam gebremst wird und nicht gegen den Übertragungskolben 65 schlägt und somit auch die Teile des Impulsgebers, insbesondere das Schwenklager 48, nicht überbeansprucht.

Beim Ausführungsbeispiel sind jeweils zwei einander diagonal gegenüberliegende Löcher 67a, 68a im Übertragungskolben 65 und in der Hülsenwand 32b vorgesehen. Zusätzlich kann in der Mantelfläche des Übertragungskolbens 65 oder in der Innenmantelfläche der Hülsenwand 32b eine Ringnut 71 vorgesehen sein, deren Hinterkante vorzugsweise mit der hinteren Begrenzungskante 67 übereinstimmt und für eine verbesserte Luftzirkulation sorgt.

Der Anschlag 66 ist beim vorliegenden Ausführungsbeispiel durch eine nach vorne weisende Schulterfläche an der Hülsenwand 32b gebildet, die mit einem vorzugsweise am vorderen Ende des Übertragungskolbens 65 angeordneten Flansch 65a zusammenwirkt.

Bei diesem Ausführungsbeispiel sind somit die Anschläge 53a, 53b durch das Dämpfungsglied 45 in Form eines Dämpferkolbens gebildet, gegen dessen vorderen, vorzugsweise radial und eben angeordneten Stirnfläche 54 die radialen Hebelenden 55a, 55b der z. B. radial und eben verlaufenden gemeinsamen Rückenfläche 56 der Impulsnehmerhebel 47a, 47b stoßen.

Die Dämpfungsvorrichtung 62 ist auch bei einem Impulsgeber in der vorbekannten Ausgestaltung mit nur einem Impulsnehmerhebel funktionsfähig und in gleicher Weise vorteilhaft.

Wie es das Ausführungsbeispiel nach Fig. 3 außerdem zeigt, kann das Schwenklager 48 in einer Vorsatzhülse 75 entsprechend ausgebildet sein, die z. B. auf die Hülsenwand 32b von vorne bis zu einer Schulterfläche 76 aufgeschraubt ist. Hierzu weisen die Vorsatzhülse 75 in ihrem hinteren Endbereich ein Innengewinde und die Hülsenwand 32b in ihrem vorderen Bereich ein Außengewinde auf. In diesem Falle ist die Stegwand 32c Teil der Vorsatzhülse 75.

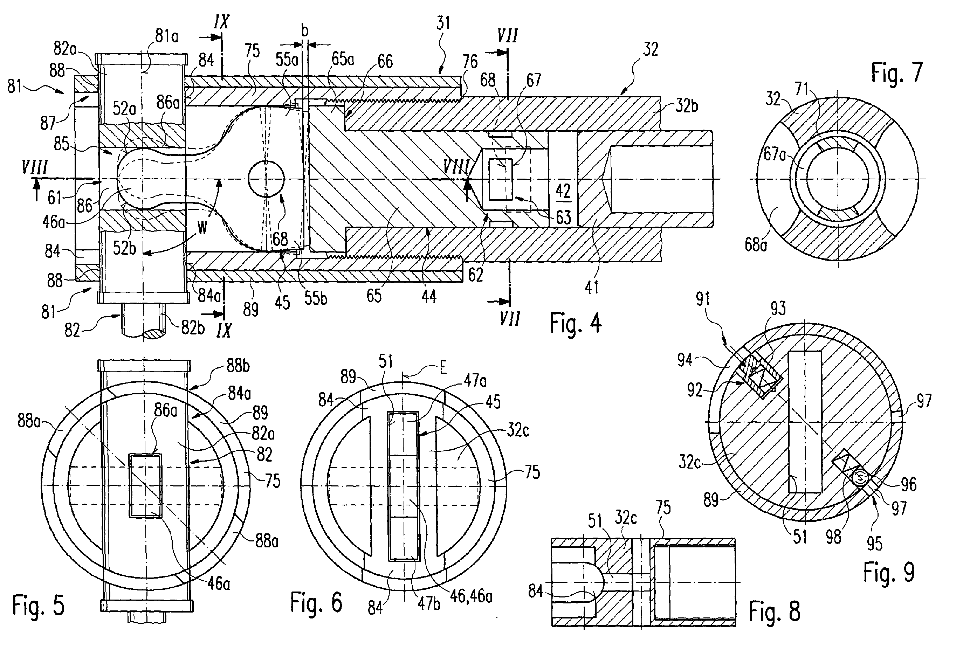

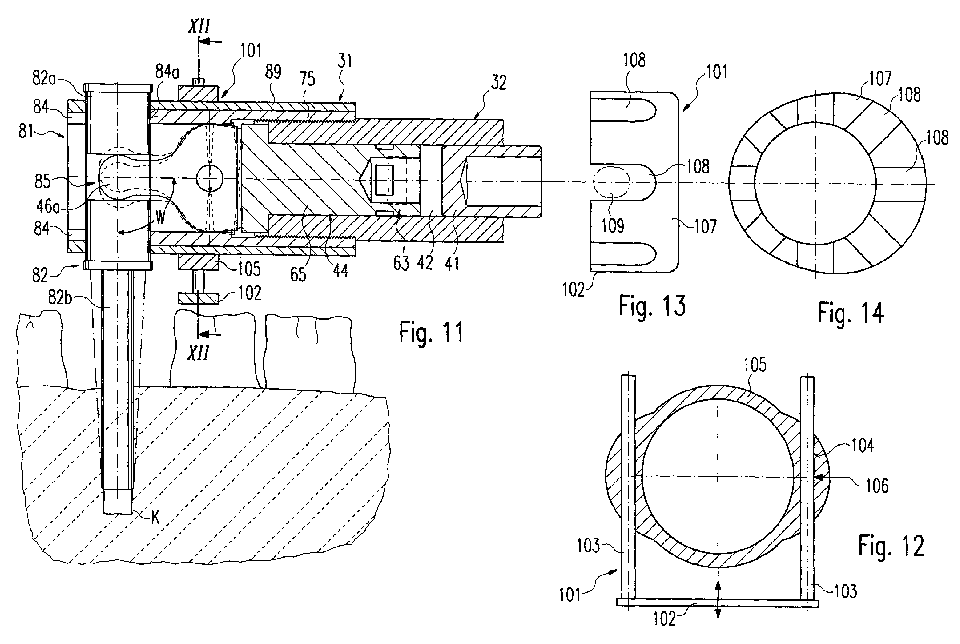

Das Ausführungsbeispiel nach Fig. 4, bei dem gleiche oder vergleichbare Teile mit gleichen Bezugszeichen versehen sind, unterscheidet sich vom Ausführungsbeispiel nach Fig. 2 oder 3 dadurch, daß am vorderen Endbereich des Handstückschaftes 32 oder der Vorsatzhülse 75 eine Haltevorrichtung 81 zum lösbaren Halten eines Werkzeugs 82 am Handstückschaft 32 vorgesehen ist. Das Werkzeug 82 ist von länglicher Bauform, wobei es sich in seiner gehaltenen Position quer zum Handstückschaft 32 erstreckt und in seiner Längsrichtung hin und her bewegbar gehalten bzw. gelagert ist. Das Werkzeug 82 weist einen vorzugsweise zylindrischen Werkzeugschaft 82a auf, an dessen einem Stirnende sich koaxial ein Arbeitsabschnitt 82b anschließt, dessen Querschnitt eine geeignete Form hat, z. B. rund sein kann, und der sich zu seinem freien Ende hin verjüngen kann, z. B. keil- oder kegelförmig (Fig. 11). Bei dem Werkzeug 82 kann es sich um ein Osteotom handeln, das dazu dient, eine Implantat-Kavität K in einem Knochen des zu behandelnden Körpers einzuarbeiten, insbesondere durch Aufweitung eines vorgefertigten Loches im Knochen. Um die Kavität zu schaffen, wird das Osteotom mit dem Impulsgeber durch einander folgende Impulsübertragungen eingetrieben. Hierbei erfüllt das Handstück 33 sowohl eine Halte-bzw. Führungsfunktion als auch eine Antriebsfunktion für das Werkzeug.

Das Werkzeug 82 ist durch eine in ihrer Gesamtheit mit 85 bezeichnete Kopplungseinrichtung mit dem Impulskopf 46a lösbar verbunden, wobei der Impulskopf 46a das handstückseitige Kopplungsteil der Kopplungseinrichtung 85 bildet. Das werkzeugseitige Kopplungsteil ist durch eine am Werkzeugschaft 32a angeordnete Kopfaufnahme 86, insbesondere in Form einer sich darin quer erstreckenden Ausnehmung, vorzugsweise rechteckiger Querschnittsform, gebildet, zwischen deren sich quer zur Werkzeugachse 82c erstreckenden Begrenzungsflächen 86a der Impulskopf 46a mit geringem Bewegungsspiel paßt. Die Kopfaufnahme 86 ist vorzugsweise nach beiden Seiten hin offen, so daß der Impulsgeber 46a wahlweise von der einen und bei verdrehter Anordnung des Werkzeugs 82 von der anderen Seite her einfassen kann. Die Breite der Kopfaufnahme 86 ist mit Bewegungsspiel an die Breite des Impulskopfes 46b angepaßt. Es können auch zwei Kopfaufnahmen 86 z. B. in Form von Ausnehmungen außen an beiden Seiten des Werkzeugschaftes 82a angeordnet sein, in die ein Impulskopf 46a in gabelförmiger Bauweise einfaßt.

Beim Ausführungsbeispiel weist die Haltevorrichtung 81 zwei stirnseitig offene Aufnahmelöcher 84 in der Hülsenwand 32b bzw. Vorsatzhülse 75 für den Werkzeugschaft 82a auf, in die dieser in der Längsrichtung des Impulskopfes 46a einsteckbar ist. Zur bezüglich des Handstückschafts 32 axialen Fixierung des Werkzeugs 82 in der Haltevorrichtung 81 sind quer vor die Aufnahmelöcher 84 und wieder zurück bewegbare Verriegelungselemente, z. B. ein Bajonettverschluß 87 mit Bajonettöffnungen 88 an einer Bajonetthülse 89 vorgesehen. Die Bajonetthülse 89 ist auf dem Werkzeugschaft 32 bzw. auf der Vorsatzhülse 75 passend aufgeschoben, drehbar gelagert und gegen eine axiale Verschiebung gesichert.

Die Bajonettöffnungen 88 sind mit einem axialen Lochabschnitt 88a und einem sich daran in Umfangsrichtung anschließenden Lochabschnitt 88b winkelförmig geformt. Die Bajonetthülse 89 ist zwischen einer Offenstellung und einer Schließstellung so in Umfangsrichtung hin und her manuell schwenkbar, daß dann, wenn die axialen Öffnungsabschnitte 88a sich in Flucht mit den Aufnahmelöchern 84 befinden, der Werkzeugschaft 82a in der Längsrichtung des Handstückschafts 32 in die Aufnahmelöcher 84 einsetzbar ist und nach einer Verdrehung der Bajonetthülse 89 in die entsprechende Umfangsrichtung in den Bereich der Lochabschnitte 88b gelangt, in denen er durch die sich vor den Aufnahmelöchern 84 erstreckenden Bajonettstege 88c axial gesichert ist. Die Aufnahmelöcher 84 und Bajonettöffnungen 88 sind in ihrer Größe so an die vorzugsweise runde Querschnittsform und -größe des Werkzeugschaftes 82 angepaßt, daß sie eine Längsführung 84a für den Werkzeugschaft 82a bilden. Die Länge des Werkzeugschaftes 82 ist so groß bemessen, daß seine Enden sich in jeder Hubstellung im Bereich der Bajonetthülse 89 bzw. der Aufnahmelöcher befinden und geführt sind. Die Haltevorrichtung 81 ist bezüglich der Längsmittelachse des Impulskopfes 46a bzw. des Handstückschaftes 32 symmetrisch ausgebildet. D. h., das Werkzeug ist wahlweise in Stellungen montierbar, in denen der Arbeitsabschnitt 82b zu der einen oder zu der anderen Seite des Impulsgebers 31 bzw. des Handstückschaftes 32, hier bezüglich der Bajonetthülse 89, absteht.

Beim Ausführungsbeispiel schließen die Längsmittelachse 32a des Handstückschaftes 32 und die Längsmittelachse 81a der Haltevorrichtung 81 bzw. des Werkzeugs einen rechten Winkel W ein. Dieser Winkel W kann in einem Winkelbereich von etwa 70 bis 120 ° liegen. D. h., das Werkzeug 82 kann mit der Längsmittelachse 32a des Handstückschaftes 32 einen spitzen oder stumpfen Winkel im vorgenannten Bereich einschließen. Diese Ausgestaltung hat den Vorteil, daß sich der Impulsgeber 31 bzw. das Handstück 33 für bestimmte Formgebungen des zu bearbeitenden Körpers, z. B. für ein Arbeiten am Unter- oder Oberkiefer, besonders gut eignet. Bei einer vorerwähnten symmetrischen Ausbildung der Haltevorrichtung 33 läßt sich darüber hinaus der Vorteil erreichen, daß durch ein Montieren des Werkzeugs 82 in einer Stellung, in der sein Arbeitsabschnitt 82b zu der einen oder zu der anderen Seite des Handstückschaftes 32 absteht, ein spitzer oder stumpfer Winkel W einstellen läßt, der sich für die jeweilige Behandlungsstelle eignet.

An den Enden des Werkzeugschaftes 82 können kleine Ringwülste 82d angeordnet sein.

Die axiale Sicherungsvorrichtung 91 für die Bajonetthülse 89 kann durch einen radialen Sicherungsstift 92 gebildet sein, der in einem Aufnahmeloch 93 im Handstückschaft 32 bzw. in der Vorsatzhülse 75 angeordnet ist und radial mit einem verjüngten Stiftzapfen nach außen in eine sich in Umfangsrichtung erstreckende Sicherungsnut 94 mit Bewegungsspiel einfaßt. Vorzugsweise ist der Sicherungsstift 92 radial einwärts verschiebbar und durch eine Feder radial nach außen vorgespannt, wobei er dadurch gegen ein Herausschieben nach außen gesichert ist, daß eine Schulterfläche einer inneren Stiftstufe durch den inneren Rand der Sicherungsnut 94 begrenzt ist. Die Sicherungsnut 94 ist so lang bemessen, daß in den Endstellungen der Bajonetthülse 89 die Bajonettöffnungen 88 in vorbeschriebener Weise offen bzw. geschlossen sind.

Aufgrund der Einfederbarkeit des Sicherungsstiftes 92 ist die axiale Sicherungsvorrichtung 91 lösbar, so daß nach einem manuellen radialen Einschieben des Sicherungsstiftes 92 die Bajonetthülse 89 vom Handstückschaft 32 abgezogen und wieder montiert werden kann.

Zur Sicherung der Bajonetthülse 89 in ihrer Offenstellung und Schließstellung ist eine Drehsicherung 95 vorgesehen, die in den Drehendstellungen jeweils im Sinne eines Druckpunktes überdrückbar ist, so daß eine ungewollte Drehung der Bajonetthülse 89 verhindert ist. Durch manuelles Überdrücken durch einen geringen Kraftaufwand sind die Druckpunkte jedoch überdrückbar. Hierzu sind vorzugsweise Verrastungsvorrichtungen mit einem elastisch nachgiebigem Verrastungselement 96 vorgesehen, das jeweils in eine Verrastungsausnehmung 97 einfederbar ist. Wie Fig. 9 zeigt, kann ein Verrastungselement 96 in Form einer Kugel oder Stiftes in einem radialen Führungsloch 98 in einer Stegwand gelagert und durch eine Feder 99 radial nach außen gegen die Verrastungsausnehmung 96 bildende Löcher in der Bajonetthülse 89 vorgespannt sein, die sich in den Drehendstellungen befinden.

Beim Ausführungsbeispiel nach Fig. 11 ist im vorderen Endbereich des Handstückschaftes 32 eine einstellbare Tiefenbegrenzungsvorrichtung 101 für das Werkzeug 82 bzw. das Osteotom vorgesehen. Mittels eines hinter dem Werkzeug 82 angeordneten Anschlagteils 102, das sich auf der Werkzeugseite des Handstückschaftes 32 befindet, ist die gewünschte Tiefe beim Setzen eines Osteotoms durch Anschlag gegen einen benachbarten Zahn oder das Zahnfleisch begrenzt. Das Anschlagteil 102 kann durch eine Leiste gebildet sein, die an ihrem einen Ende oder an beiden Enden an einem Führungsstift 103 befestigt ist, der in einem Führungsloch 104 eines Halteringes 105 längs verschiebbar und durch ein Sicherungselement 106, z. B. eine Schraube, wahlweise feststellbar ist. Beim Ausführungsbeispiel weist der Haltering 105 auf beiden Seiten zwei Führungslöcher 104 in seitlichen Materialverdickungen auf.

Beim Ausführungsbeispiel nach Fig. 13 ist das Anschlagteil 102 durch die Umfangsfläche eines exzentrischen Anschlagringes 107 gebildet, der auf dem Handstückschaft 32 bzw. auf der Bajonetthülse 89 drehbar gelagert ist und auf dem Umfang verteilt axial offene Ausnehmungen 108 aufweist, mit denen er zu seiner Drehsicherung auf einen Sicherungszapfen 109 axial aufsteckbar ist, der radial vom Handstückschaft 32 bzw. von der Bajonetthülse 89 absteht.

## Patentansprüche

1. Maschineller Impulsgeber (31) für dentalmedizinische oder medizinische Zwecke mit einem beweglichen Impulskopf (46a), der bei Auslösung eines Impulses von einer Bereitschaftsposition schlagartig in eine bestimmte Wirkrichtung (R1, R2) bewegbar ist,
**dadurch gekennzeichnet,**
**daß** der Impulskopf (46a) bei Auslösung eines Impulses ebenso schlagartig in die entgegengesetzte Richtung (R1, R2) bewegbar ist, wobei die Richtung (R1, R2) vorher einstellbar ist.

2. Maschineller Impulsgeber nach Anspruch 1,
**gekennzeichnet durch**
eine Voreinstellvorrichtung (61) für den Impulskopf (46a), **durch** die der Impulskopf (46a) nach Voreinstellung in die eine Wirkrichtung (R1, R2) bei Auslösung des Impulses in die andere Wirkrichtung (R1, R2) bewegt wird.

3. Maschineller Impulsgeber nach Anspruch 2, wobei der Impulskopf (46a) um eine Schwenkachse (48a) bewegbar und die Schwenkbewegung mittels eines Schlagkolbens (41) auslösbar ist, wenn der Schlagkolben (41) auf einen an dem Impulskopf (46a) vorgesehenen Auftreffbereich (55a, 55b) auftrifft,
**dadurch gekennzeichnet,**
**daß** der Impulskopf (46a) einen zweiten Auftreffbereich (55a, 55b) für den Schlagkolben (41) aufweist, der - von dem Schlagkolben (41a) aus gesehen - in Bezug auf die Schwenkachse (48a) gegenüberliegend zu dem ersten Auftreffbereich angeordnet ist, wodurch der Impulskopf (46a) eine um die Schwenkachse (48a) schwenkbare Wippe bildet, so daß sich der Impulskopf (46a) in die eine oder die andere Richtung schwenkt, je nachdem, welcher Auftreffbereich (55a, 55b) näher am Schlagkolben (41) ist, bevor dieser auf den Impulskopf (46a) auftrifft.

4. Maschineller Impulsgeber nach Anspruch 3,
**dadurch gekennzeichnet,**
**daß** der Impulskopf (46a) ein T-förmiges Bauteil ist mit einem sich nach vorne erstreckenden Impulsgeberhebel (46), an dessen freiem Ende der Impulskopf (46a) angeordnet ist, und mit zwei sich vom hinteren Ende des Impulsgeberhebels (46) quer zu einander gegenüberliegenden Seiten hin erstreckenden Impulsnehmerhebeln (47a, 47b), die mit den Rückenflächen ihrer rückseitigen Hebelenden die Auftreffbereiche (55a, 55b) bilden.

5. Maschineller Impulsgeber nach Anspruch 3 oder 4,
**dadurch gekennzeichnet,**
**daß** die Schwenkachse (48a) die Längsmittelachse (32a) des Impulsgebers (31) im wesentlichen schneidet.

6. Maschineller Impulsgeber nach Anspruch 4 oder 5,
**dadurch gekennzeichnet,**
**daß** der Impulskopf (46a) in seiner Bereitschaftsstellung über seine Mittelposition hinaus geschwenkt ist.

7. Maschineller Impulsgeber nach Anspruch 6,
**dadurch gekennzeichnet,**
**daß** die Schwenkbewegungen des Impulskopfes (46a) in die Wirkrichtungen (R1, R2) durch die Bereitschaftspositionen bestimmende Anschläge (53a, 53b) begrenzt sind.

8. Maschineller Impulsgeber nach Anspruch 7,
**dadurch gekennzeichnet,**
**daß** die Impulsnehmerhebel (47a, 47b), vorzugsweise mit ihren Auftreffbereichen (55a, 55b), mit den Anschlägen (53a, 53b) zusammenwirken.

9. Maschineller Impulsgeber nach einem der vorherigen Ansprüche,
**dadurch gekennzeichnet,**
**daß** der Impulskopf (46a) in der Schwenkebene (E) verdickt ist und vorzugsweise diese Verdickung gerundete oder zylinderabschnittförmige Impulskopfflächen (52a, 52b) aufweist.

10. Maschineller Impulsgeber (31) für dentalmedizinische oder medizinische Zwecke mit einem beweglichen Impulskopf (46a), der bei Auslösung eines Impulses von einer Bereitschaftsposition schlagartig in eine bestimmte Wirkrichtung (R1, R2) bewegbar ist, oder Impulsgeber (31) nach einem der vorherigen Ansprüche,
**dadurch gekennzeichnet,**
**daß** eine Dämpfungsvorrichtung (62) vorgesehen ist, die das Auftreffen des Schlagkolbens (41) dämpft oder verhindert, wenn der Impulskopf (46a) sich nicht in seiner Bereitschaftsposition befindet.

11. Maschineller Impulsgeber nach einem der Ansprüche 3 bis 10,
**dadurch gekennzeichnet,**
**daß** dem Schlagkolben (41) ein Führungskanal (42) vorgeordnet ist, in dem er durch Druckluft in Richtung auf die Auftreffbereiche (55a, 55b) bewegbar ist.

12. Maschineller Impulsgeber nach Anspruch 11,
**dadurch gekennzeichnet,**
**daß** die Dämpfungsvorrichtung (62) ein Ventil aufweist, das eine den Führungskanal (42) mit der Umgebung verbindende Luftöffnung (63) sperrt bzw. drosselt.

13. Maschineller Impulsgeber nach Anspruch 12,
**dadurch gekennzeichnet,**
**daß** das Ventil einen Ventilschieber aufweist, der durch die Schwenkbewegung oder die Schwenkbewegungen des oder der Impulsnehmerhebel (47a, 47b) zwischen seiner Offenstellung und seiner Drossel- bzw. Sperrstellung steuerbar ist.

14. Maschineller Impulsgeber nach Anspruch 13,
**dadurch gekennzeichnet,**
**daß** hinter dem oder den Auftreffbereichen (55a, 55b) ein den Ventilschieber bildender Übertragungskolben (65) im Führungskanal (62) durch die Schwenkbewegung oder die Schwenkbewegungen der Auftreffbereiche (55a, 55b) axial verschiebbar gelagert ist.

15. Maschineller Impulsgeber nach einem der Ansprüche 12 bis 14,
**dadurch gekennzeichnet,**
**daß** das Ventil durch die Luftöffnung (63) in der Wandung (32b) des Führungskanals (42) begrenzende Begrenzungskanten (67, 68) an der Wandung (32b) und am Übertragungskolben (65) gebildet ist.

16. Maschineller Impulsgeber (31) für dentalmedizinische oder medizinische Zwecke mit einem beweglichen Impulskopf (46a), der bei Auslösung eines Impulses von einer Bereitschaftsposition schlagartig in eine bestimmte Wirkrichtung (R1, R2) bewegbar ist, oder Impulsgeber (31) nach einem der vorherigen Ansprüche,
**dadurch gekennzeichnet,**
**daß** an dem den Impulskopf (46a) lagernden Körper des Impulsgebers (31) eine Haltevorrichtung (81) zum lösbaren Montieren eines Werkzeugs (82) vorgesehen ist, wobei die Haltevorrichtung (81) eine sich längs der oder den Wirkrichtungen (R1, R2) erstreckende Führung (84a) für das Werkzeug (82) bildet und der Induktionskopf (46a) Teil einer zwischen dem Impulskopf (46a) und dem Werkzeug (82) wirksamen Koppeleinrichtung (85) zum Übertragen des Impulses auf das Werkzeug (82) ist.

17. Maschineller Impulsgeber nach Anspruch 16,
**dadurch gekennzeichnet,**
**daß** der Körper des Impulsgebers (31) sich nach vorne bis in den Bereich des Impulskopfes (46a) erstreckt.

18. Maschineller Impulsgeber nach Anspruch 16 oder 17,
**dadurch gekennzeichnet,**
**daß** die Haltevorrichtung (81) zwei bezüglich des Impulskopfes (46a) seitlich in der Schwenkebene (E) einander gegenüberliegende Aufnahmelöcher (84) im Körper des Impulsgebers (31) für das Werkzeug (82) aufweist.

19. Maschineller Impulsgeber nach Anspruch 18,
**dadurch gekennzeichnet,**
**daß** die Aufnahmelöcher (84) vorderseitig offen sind und das Werkzeug (82) durch eine auf dem Körper drehbar gelagerte Hülse (89) mit Bajonettausnehmungen (89) zum Sichern des Werkzeugs in den Aufnahmelöchern (84) vorgesehen ist.

20. Maschineller Impulsgeber nach einem der Ansprüche 16 bis 19,
**dadurch gekennzeichnet,**
**daß** das Werkzeug (82) durch ein Osteotom mit einem Schaft (82a) gebildet ist, in dem eine quer zugängliche Kopfaufnahme (86) für den Impulskopf (46a) angeordnet ist.
